# EUROPEAN PATENT APPLICATION

(11) **EP 4 595 765 A1**
(43) Date of publication of application: **06.08.2025**
(21) Application number: 24305169.5
(22) Date of filing: 31.01.2024
(51) Int. Cl.: A23F 5/48, A61Q 19/00, B01D 11/02

(54) **COFFEE BUTTER, METHOD OF PREPARATION AND USES THEREOF**

(71) Applicant: Cabeco, 59262 Sainghin-en-Melantois (FR)
(72) Inventor: PERRY, David, 59262 SAINGHIN EN MELANTOIS (FR); VERWILGHEN, Frederic, 1200 WOLUWE-SAINT-LAMBERT (BE)
(74) Representative: Plasseraud IP

(57) **Abstract**

The present invention provides a method for processing spent coffee grounds using supercritical fluid extraction. This method provides a solid lipidic extract, which presents wound healing, antibacterial and skin moisturizing properties, and a delipidated coffee powder that can be micronized into a very fine powder making it suitable for use as pigments in cosmetics, inks or paints.

## Description

### Technological Background

Coffee is a circum-equatorial crop, in majority grown in Brazil, Mexico and Vietnam, but also cultivated in many other countries in the Americas, Africa and Asia. The coffee fruits are harvested from the *Coffea* plant, mainly from the *arabica* and *robusta* species. The coffee fruits undergo a pre-processing in order to remove the layers around the coffee bean, which yields coffee green beans. Coffee green beans are then roasted before being processed into a beverage.

Instant coffee is produced from ground roasted coffee through a stepwise process, wherein the water-soluble fraction is extracted from the ground roasted coffee and then dried to yield the instant coffee.

As a by-product of this extraction process, large amounts of spent coffee grounds (SCG) are obtained.

Upcycling of SCG is therefore of major interest. For example, recovery and upcycling of the lipids contained in SCG has been a focus of research. Applications of lipidic extracts from SCG as biodiesel or as bioplastics have been investigated. However, the direct use of pure lipidic extracts from SCG in cosmetics or for therapeutic applications has not yet been reported.

To extract the lipids from SCG, different extraction methods have been employed, such as screw pressing, organic solvent extraction, enzymatic assisted extraction, or super-critical extraction. However, up until now no process has proved adequate for yielding lipidic extracts directly useable for cosmetic or therapeutic applications. Indeed, the current extraction processes yield low amounts of lipid fractions, that are dark colored, smelly, and liquid. Concomitantly, the delipidated coffee powders obtained are of low commercial value, in particular because they still contain relatively large amounts of lipids.

Specifically, no processes are reported that allow direct obtention of pure solid coffee butters of a pleasing and appropriate color, for incorporation into high value products for cosmetics, medicines, and food or for use in any industrial sector. Up until now, none have led to the obtention of high value, high quality pure solid butters on one hand and high value delipidated coffee powders within the same process. Currently, all commercial coffee butters are mixtures of coffee oil and solid vegetable oils or fats.

An object of the present disclosure thus concerns a process allowing the direct obtention of solid pure coffee butters. At the same time, this process yields high value delipidated coffee powders that can be further processed to sizes of below 50 µm, making them useful as pigments in cosmetics, or in inks and paints.

Further, another object of the present disclosure concerns solid lipidic extracts from spent coffee grounds (for example, the lipidic extracts obtained by the process of the invention). The lipidic extract of the invention presents advantageous properties for cosmetics or for therapeutical applications. In particular, it was found that the lipidic extract of the invention presents very advantageous skin moisturizing, wound healing, and bactericidal properties.

### Summary of the invention

The present invention concerns the following items:
Item 1: Method for processing spent coffee grounds comprising the following steps:
   - Providing dry spent coffee grounds having a moisture content of below 5% w/w, preferably below 4% w/w, more preferably below 3% w/w, even more preferably below 2% w/w, the moisture content being expressed relative to the total weight of the dry spent coffee grounds;
   - Extracting lipids from the dry spent coffee grounds using supercritical fluid extraction, using, as the supercritical fluid, a fluid comprising at least 90% w/w CO₂ relative to the total weight of the fluid, to obtain a lipidic extract and a delipidated coffee powder, wherein the supercritical fluid extraction is performed in an apparatus comprising an extraction cell and wherein the supercritical fluid extraction is performed with the following parameters:
      ∘ a pressure inside the extraction cell of 200 to 300 bar;
      ∘ a temperature inside the extraction cell of 35 to 70 °C;
      ∘ a CO₂ flowrate of 10 to 50 kg/h;
      ∘ a solvent-to-feed ratio of above 20 kg CO₂/kg of dry spent coffee grounds;
   - recovering the delipidated coffee powder and the lipidic extract;
   wherein the dry spent coffee grounds are of industrial instant coffee production origin.
Item 2: The method of item 1, wherein providing the dry spent coffee grounds comprises providing wet spent coffee grounds and drying the wet spent coffee grounds at a temperature of 110°C to 150°C, preferably of 120°C to 140°C, more preferably of 125°C to 135°C, for 2h to 4h.
Item 3: The method of item 1 or 2, wherein the dry spent coffee grounds consist of particles having a particles size distribution such that all the particles have a size below 3 mm, preferably below 2 mm, more preferably wherein all the particles have a size below 2 mm and less than 30% w/w of the particles have a size of 1 mm to 2 mm; and wherein at least 30% w/w of the particles have a size above 250 µm, the particles weight percentages being expressed relative to the total weight of the dry spent coffee grounds.
Item 4: The method of item 3, wherein providing the dry spent coffee grounds comprises providing coarse spent coffee grounds and grinding the coarse spent coffee grounds, where applicable the wet spent coffee grounds are the coarse spent coffee grounds and the grinding of the coarse spent coffee grounds is performed before or after the drying of the wet spent coffee grounds, preferably after the drying, preferably after the drying.
Item 5: The method of any one of items 1 to 4, wherein the fluid comprises more than 99% w/w CO₂ relative to the total weight of the fluid, preferably is pure CO₂.
Item 6: The method of any one of items 1 to 5, wherein the solvent-to-feed ratio is of 20 to 60, preferably of 20 to 40, more preferably of 20 to 30 kg CO₂/kg of dry spent coffee grounds.
Item 7: The method of any one of items 1 to 6, further comprising a step of micronizing the delipidated coffee powder to a particle size of below 50 µm as measured by laser diffraction.
Item 8: Delipidated coffee powder obtainable by the method of any one of items 1 to 7.
Item 9: Delipidated coffee powder having a lipid content of below 6% w/w, for example of 2% w/w to 6% w/w, relative to the total weight of the delipidated coffee powder.
Item 10: Lipidic extract obtainable by the method of any one of items 1 to 7.
Item 11: A solid lipidic extract from spent coffee grounds having a melting temperature of at least 20°C, preferably of 25°C and 30°C.
Item 12: The lipidic extract of item 10 or 11, comprising at least 70% w/w, preferably at least 80% w/w of lipids relative to the total weight of the lipidic extract.
Item 13: The lipidic extract of any one of items 10 to 12 for use in wound healing.
Item 14: The lipidic extract of any one of items 10 to 12 for use in therapeutic treatment of a bacterial infection.
Item 15: The lipidic extract of any one of items 10 to 12 for use in therapeutic skin moisturizing.
Item 16: A bandage comprising the lipidic extract of any one of items 10 to 15.
Item 17: A non-therapeutic method of disinfecting a surface, the method comprising the step of contacting the surface with the lipidic extract of any one of items 10 to 12.
Item 18: Use of the lipidic extract of any one of items 10 to 12 in non-therapeutic skin moisturizing care.

### Detailed description

The present invention provides a method for processing spent coffee grounds comprising the following steps:
- Providing dry spent coffee grounds having a moisture content of below 5% w/w, preferably below 4% w/w, more preferably below 3% w/w, even more preferably below 2% w/w, the moisture content being expressed relative to the total weight of the dry spent coffee grounds;
- Extracting lipids from the dry spent coffee grounds using supercritical fluid extraction, using, as the supercritical fluid, a fluid comprising at least 90% w/w CO₂ relative to the total weight of the fluid, to obtain a lipidic extract and a delipidated coffee powder, wherein the supercritical fluid extraction is performed in an apparatus comprising an extraction cell and wherein the supercritical fluid extraction is performed with the following parameters:
   ○ a pressure inside the extraction cell of 200 to 300 bar;
   ○ a temperature inside the extraction cell of 35 to 70 °C;
   ○ a CO₂ flowrate of 10 to 50 kg/h;
   ○ a solvent-to-feed ratio of above 20 kg CO₂/kg of dry spent coffee grounds;
- recovering the delipidated coffee powder and the lipidic extract;
wherein the dry spent coffee grounds are of industrial instant coffee production origin.

The method of the invention unexpectedly allows to obtain solid lipidic extracts from spent coffee grounds. The use of a supercritical fluid extraction method, the appropriate control of the moisture content of the spent coffee grounds used as starting material combined with their industrial origins allows to obtain a solid lipidic extract having a melting temperature of at least 20°C, for example of 25°C to 30°C. Methods of the prior art using either poorly dried spent coffee grounds or spent coffee grounds obtained, for example from coffee shops or bars, or using other techniques than supercritical fluid extraction yielded liquid oils, sometimes smelly or with a dark color, and generally in poor yields.

As defined herein, spent coffee grounds are of industrial instant coffee production origin when they are the by-product of a process for the industrial production of instant coffee. This is as opposed, for example, to the spent coffee grounds that can be obtained from coffee shops or bars or individuals brewing their own coffee. As defined herein, spent coffee grounds of industrial instant coffee production origin comprise the spent coffee grounds that are directly obtained by the industrial instant coffee production process and such spent coffee grounds that have been further dried and/or ground after the industrial instant coffee production process. Industrial extraction processes used to produce instant coffee yield spent coffee grounds of a different quality than coffee brewing in bars or coffee shops and this contributes to the unexpected results obtained with the method of the invention.

The lipidic extract obtained by the method of the invention has a K value in the CMYK color model that is lower than 60 and a C value that is lower than 10. This corresponds to a clearer color than the previously available lipidic extracts from SCG. Moreover, the lipidic extracts of the present invention have a more acceptable smell.

The K and C values of the lipidic extract in the CMYK color model are measured using the color eyedropper tool in the CorelDRAW software on pictures of the lipidic extract.

Providing the dry spent coffee grounds may comprise providing wet spent coffee grounds and drying the wet spent coffee grounds at a temperature of 110°C to 150°C, preferably of 120°C to 140°C, more preferably of 125°C to 135°C, for 2h to 4h.

It was found that, with the above temperatures, it was possible to dry efficiently the spent coffee grounds without denaturing the lipids or overcooking the spent coffee grounds.

The expressions "dry spent coffee grounds" and "wet spent coffee grounds" are used to differentiate the drier spent coffee grounds from the wetter spent coffee grounds. They are not indicating a specific value of the moisture content other than the ones mentioned explicitly. When wet coffee grounds are mentioned, it should be understood that the dry spent coffee grounds are the product of the drying of the wet spent coffee grounds.

Since the dry spent coffee grounds are of industrial instant coffee production origin, it should be understood that the wet spent coffee grounds are also of industrial instant coffee production origin.

Preferably, the dry spent coffee grounds consist of particles having a particles size distribution such that all the particles have a size below 3 mm, preferably below 2 mm, more preferably wherein all the particles have a size below 2 mm and less than 30% w/w of the particles have a size of 1 mm to 2 mm; and wherein at least 30% w/w of the particles have a size above 250 µm, the particles weight percentages being expressed relative to the total weight of the dry spent coffee grounds.

Where the granulometry of spent coffee grounds directly obtained by industrial instant coffee production processes do not have the above granulometry, it is possible to adjust said granulometry via a grinding step. Said grinding step may be performed using instruments and methods known of the skilled person, for example with a cutting mill equipped with a sieve. Providing the dry spent coffee grounds then comprises providing coarse spent coffee grounds and grinding the coarse spent coffee grounds, where applicable the wet spent coffee grounds may be the coarse spent coffee grounds (the spent coffee grounds are initially coarser and wetter than the dry spent coffee grounds used in the extraction step) and the grinding of the coarse spent coffee grounds may happen before or after the drying of the wet spent coffee grounds. In this case, the intermediate product is respectively fine and wet or coarse and dry.

The expression "coarse spent coffee grounds" is used to differentiate the coarser spent coffee grounds that are ground from the finer dry spent coffee grounds used in the extracting step. It is not indicating a specific granulometry.

Particles sizes above 0.08 mm are measured by sieving, using sieves of decreasing aperture sizes (sieve analysis). The sieves used are metal wire cloth according to standard ISO 3310-1:2016, so that the apertures are square.

Preferably, the fluid comprises more than 99% w/w CO₂, preferably is pure CO₂.

Preferably, the solvent-to-feed ratio is of 20 to 60, more preferably of 20 to 40, even more preferably of 20 to 30 kg CO₂/kg of dry spent coffee grounds.

Although, using higher solvent-to-feed ratio allows a more complete extraction of the lipids, it was found that a solvent-to-feed ratio of 20 to 40, or even 20 to 30 kg CO₂/kg of spent coffee grounds was optimal as going above these values only marginally improved the yield of extraction while significantly increasing the cost of the extraction.

The method of the invention may further comprise a step of micronizing the delipidated coffee powder to a particle size of below 50 µm as measured by laser diffraction.

Micronization is preferably performed by wet ultrasonication, wherein the cavitation caused by sonication in a slurry of the particles causes the breaking of the particles in the slurry and therefore their reduction in size.

Laser diffraction measurements are performed according to standard ISO 13320 (2020).

The present invention provides the products obtainable by the method of the invention, namely the delipidated coffee powder and the lipidic extract.

The present invention provides delipidated spent coffee grounds (herein also called delipidated coffee powders) having a lipid content of below 6% w/w, for example of 2% w/w to 6% w/w, relative to the total weight of the delipidated coffee powder. Such a delipidated coffee powder may be easily micronized to particles size of below 50 µm and as low as 10 µm, thanks to the quality of the lipid extraction. The low particle sizes obtained after micronization make it possible to use the micronized delipidated coffee powder as pigment particles, for example in cosmetic products, in inks or in paints.

The delipidated coffee powders obtained by the extraction processes of the prior art have a higher lipid content than the delipidated coffee powder of the present invention. Only the low lipid contents obtained with the present invention allow efficient micronization of the delipidated powders.

As defined herein, delipidated coffee powder is a product obtained from spent coffee grounds as the only starting material, for example obtained from spent coffee grounds of industrial instant coffee production origin.

The present invention provides for the first time to solid lipidic extracts from spent coffee grounds, which may be used directly in cosmetic or therapeutic applications. For example, a solid lipidic extract from spent coffee grounds having a melting temperature of at least 20°C, preferably of between 25°C and 30°C.

As defined herein, a lipidic extract comprises at least 70% w/w of lipids relative to the total weight of the extract, preferably at least 80% w/w.

Lipids may comprise fatty acids and their derivatives (including tri-, di-, monoglycerides, and phospholipids), and sterol lipids.

As defined herein, an extract from spent coffee grounds is a product obtained from spent coffee grounds as the only starting material, for example obtained from spent coffee grounds of industrial instant coffee production origin. For example, according to the present disclosure, a mixture of lipids extracted from spent coffee grounds with another lipidic product (e.g. shea butter) is not an extract from spent coffee grounds.

Moreover, it should be understood that an extract from spent coffee grounds is the product directly obtained by the extraction process. For example, according to the present disclosure, a lipidic extract from spent coffee grounds that is modified after the extraction by an additional step of interesterification or hydrogenation is not a lipidic extract from spent coffee grounds.

Current processes for extracting the lipids from SCG only yield relatively low viscosity liquid oils. However, with the present invention, it is possible to obtain a solid lipidic extract, without the need for additional transformation or for mixing with other products.

Moreover, the lipidic extracts of the present invention have a clearer color than the previously available lipidic extracts from SCG and a more acceptable smell.

For example, the lipidic extract of the invention may have K value in the CMYK color model that is lower than 60 and a C value that is lower than 10.

The K and C values of the lipidic extract in the CMYK color model are measured using the color eyedropper tool in the CorelDRAW software on pictures of the lipidic extract.

Creamy or butter-like products are in demand in the cosmetic industry or for therapeutic applications as they are preferred by the consumer and easier to apply consistently compared to liquid products. The presently disclosed solid lipidic extracts can be used directly in cosmetic or therapeutic creamy or butter-like products, which was not the case with previously available lipidic extracts from spent coffee grounds.

It has been found that the lipidic extract of the invention has advantageous wound healing properties. In particular, *ex vivo* tests on human living skin explants where a lesion is performed using a biopsy punch show that the lipidic extract of the invention causes an increase of the proliferation of the wound buds (neo-epidermis) as well as an increase in their adhesion to the dermis.

The present invention thus also provides the lipidic extract of the invention for use in wound healing.

It has also been found that the lipidic extract of the invention has advantageous anti-bacterial properties. In particular, a bactericidal activity on *Staphylococcus aureus* has been demonstrated.

The present invention thus also provides the lipidic extract of the invention for use in therapeutic treatment of a bacterial infection.

It should be understood that the treatment of a bacterial infection can be prophylactic, i.e. the bacterial infection has not necessarily happened at the date of treatment.

The lipidic extract of the invention may also be used in non-therapeutic methods based on its anti-bacterial properties, for example, in a non-therapeutic method of disinfecting a surface, the method comprising the step of contacting the surface with the lipidic extract.

It should be understood that, when a composition comprising the lipidic extract of the invention is in contact with a surface, then the lipidic extract of the invention is also in contact with said surface.

It has also been found that the lipidic extract of the invention has advantageous skin moisturizing properties. In particular, *ex vivo* tests on human living skin explants show that the lamination aspect of the stratum corneum increases when the explant is treated with the lipidic extract of the invention. This is characteristic of a well hydrated stratum corneum. Further immunostaining of aquaporin-3 (AQP3) on the treated explants show a decrease of AQP3 upon treatment, which reflects an improvement of the barrier function of the stratum corneum and therefore an epidermis that remains well hydrated.

The present invention thus also provides the lipidic extract of the invention for use in therapeutic skin moisturizing as well as the use of the lipidic extract of the invention in non-therapeutic skin moisturizing care.

A bandage, for example an adhesive bandage, comprising the lipidic extract of the invention may be used for all of these applications.

### Example of an extraction process according to the invention

### Pre-processing

Spent coffee grounds (SCG) of industrial instant coffee production origin were ground and then dried before being fed in the extraction machine.

The grinding was performed with a cutting mill SM 300 (Retsch) using a bottom sieve with an aperture size of 1 mm. The particles size distribution of the ground SCG was measured using sieves of decreasing aperture sizes. It was determined that:
- no particles had a size above 2 mm (i.e. no particles were retained when the aperture size of the sieve was 2 mm);
- 24.5% of the particles had a size between 2 mm and 1 mm;
- 23% of the particles had a size between 1 mm and 0.8 mm;
- 26.8% of the particles had a size between 0.8 mm and 0.5 mm;
- 12.5% of the particles had a size between 0.5 mm and 0.25 mm;
- 10.9% of the particles had a size between 0.25 mm and 0.125 mm;
- 2.3% of the particles had a size below 0.125 mm.

The percentages are given by weight relative to the total weight of the SCG.

The drying was performed at 125°C for 3h until a moisture content of below 4% was achieved.

### Supercritical fluid extraction

The dried and ground SCG were loaded in the extraction cell of the extraction machine. The volume of the extraction cell was 5 L and the mass of the dried and ground SCG was 2 kg. Pure CO₂ was used as the extraction fluid.

The following extraction parameters were used (two experiments 1 and 2 with two different solvent-to-feed ratios): CO₂ flow rate: 50 kg/h
Extraction cell temperature: 40°C
Time of extraction: 60 min (experiment 1) or 120 min (experiment 2)
Solvent-to-feed ratio: 25 kg CO₂/kg SCG (experiment 1) or 50 kg CO₂/kg SCG (experiment 2)
Yield: 62% (experiment 1) or 70% (experiment 2)

### Products obtained

For both experiments (1 and 2):
- the recovered lipidic extract was an orange solid product with a melting point of 25.3°C and a lipid content of more than 80% w/w relative to the total mass of the lipidic extract, and
- the delipidated coffee powder had a lipid content of 3.99% w/w relative to the total mass of the delipidated coffee powder.

## Claims

1. Method for processing spent coffee grounds comprising the following steps:
∘ Providing dry spent coffee grounds having a moisture content of below 5% w/w, preferably below 4% w/w, more preferably below 3% w/w, even more preferably below 2% w/w, the moisture content being expressed relative to the total weight of the dry spent coffee grounds;
∘ Extracting lipids from the dry spent coffee grounds using supercritical fluid extraction, using as the supercritical fluid a fluid comprising at least 90% w/w CO₂ relative to the total weight of the fluid, to obtain a lipidic extract and a delipidated coffee powder, wherein the supercritical fluid extraction is performed in an apparatus comprising an extraction cell and wherein the supercritical fluid extraction is performed with the following parameters:
▪ a pressure inside the extraction cell of 200 to 300 bar;
▪ a temperature inside the extraction cell of 35 to 70 °C;
▪ a CO₂ flowrate of 10 to 50 kg/h;
▪ a solvent-to-feed ratio of above 20 kg CO₂/kg of dry spent coffee grounds;
∘ recovering the delipidated coffee powder and the lipidic extract;
wherein the dry spent coffee grounds are of industrial instant coffee production origin.

2. The method of claim 1, wherein providing the dry spent coffee grounds comprises providing wet spent coffee grounds and drying the wet spent coffee grounds at a temperature of 110°C to 150°C, preferably of 120°C to 140°C, more preferably of 125°C to 135°C, for 2h to 4h.

3. The method of claim 1 or 2, wherein the dry spent coffee grounds consist of particles having a particles size distribution such that all the particles have a size below 3 mm, preferably below 2 mm, more preferably wherein all the particles have a size below 2 mm and less than 30% w/w of the particles have a size of 1 mm to 2 mm; and wherein at least 30% w/w of the particles have a size above 250 µm, the particles weight percentages being expressed relative to the total weight of the dry spent coffee grounds.

4. The method of claim 3, wherein providing the dry spent coffee grounds comprises providing coarse spent coffee grounds and grinding the coarse spent coffee grounds, where applicable the wet spent coffee grounds are the coarse spent coffee grounds and the grinding of the coarse spent coffee grounds is performed before or after the drying of the wet spent coffee grounds, preferably after the drying.

5. The method of any one of claims 1 to 4, wherein the fluid comprises more than 99% CO₂, preferably is pure CO₂.

6. The method of any one of claims 1 to 5, wherein the solvent-to-feed ratio is of 20 to 60, preferably of 20 to 40, more preferably of 20 to 30 kg CO₂/kg of spent coffee grounds.

7. The method of any one of claims 1 to 6, further comprising a step of micronizing the delipidated coffee powder to a particle size of below 50 µm.

8. Delipidated coffee powder having a lipid content of below 6% w/w, for example of 2% w/w to 6% w/w, relative to the total weight of the delipidated coffee powder.

9. A solid lipidic extract from spent coffee grounds having a melting temperature of at least 20°C, preferably of 25°C to 30°C.

10. The lipidic extract of claim 9 for use in wound healing.

11. The lipidic extract of claim 9 for use in therapeutic treatment of a bacterial infection.

12. The lipidic extract of claim 9 for use in therapeutic skin moisturizing.

13. A bandage comprising the lipidic extract of any one of claims 9 to 12.

14. A non-therapeutic method of disinfecting a surface, the method comprising the step of contacting the surface with the lipidic extract of claim 9.

15. Use of the lipidic extract of claim 9 in non-therapeutic skin moisturizing care.
